# EUROPEAN PATENT SPECIFICATION

(11) **EP 4 510 902 B1**
(45) Date of publication and mention of the grant of the patent: **28.10.2026**
(21) Application number: 23791440.3
(22) Date of filing: 20.04.2023
(51) Int. Cl.: A61B 1/00, A61B 1/04, A61B 1/06, A61B 34/37, A61B 34/00

(54) **MAGNETIC ANCHORED AND ACTUATED SYSTEM AND MANUFACTURING METHOD THEREOF**
MAGNETISCHES VERANKERTES UND BETÄTIGTES SYSTEM UND HERSTELLUNGSVERFAHREN DAFÜR
SYSTÈME MAGNÉTIQUE ANCRÉ ET ACTIONNÉ ET SON PROCÉDÉ DE FABRICATION

(30) Priority: 20.04.2022 US 202263333099 P
(43) Date of publication of application: 26.02.2025
(73) Proprietor: Multi-Scale Medical Robotics Center Limited, Hong Kong (HK)
(72) Inventor: LI, Zheng, N.T. HONG KONG (HK); LI, Jixiu, N.T. HONG KONG (HK); ZHANG, Tao, N.T. HONG KONG (HK); CHENG, Truman, N.T. HONG KONG (HK); NG, Sze Hang Calvin, N.T. HONG KONG (HK); CHIU, Wai Yan Philip, N.T. HONG KONG (HK)
(74) Representative: Bryn Aarflot AS
(86) International application number: PCT/IB2023/054019
(87) International publication number: WO 2023/203510

(56) References cited:
- EP-A1- 3 797 670
- WO-A1-2012/035157
- CN-A- 103 181 748
- CN-A- 108 245 122
- CN-A- 113 017 542
- US-A1- 2010 001 592
- US-A1- 2018 084 975
- US-A1- 2021 137 622
- XU DONG ET AL: "Design and Implementation of A Magnetic Actuated Capsule Camera Robot System for Single Incision Laparoscopic Surgery*", 2020 IEEE 18TH INTERNATIONAL CONFERENCE ON INDUSTRIAL INFORMATICS (INDIN), IEEE, vol. 1, 20 July 2020 (2020-07-20), pages 225 - 230, XP033923564, DOI: 10.1109/INDIN45582.2020.9442197
- LEONG FLORENCE ET AL: "Magnetic Surgical Instruments for Robotic Abdominal Surgery", IEEE REVIEWS IN BIOMEDICAL ENGINEERING, vol. 9, 16 September 2016 (2016-09-16), pages 66 - 78, XP011623620, ISSN: 1937-3333, [retrieved on 20160920], DOI: 10.1109/RBME.2016.2521818
- DI NATALI C ET AL: "Trans-abdominal Active Magnetic Linkage for robotic surgery: Concept definition and model assessment", ROBOTICS AND AUTOMATION (ICRA), 2012 IEEE INTERNATIONAL CONFERENCE ON, IEEE, 14 May 2012 (2012-05-14), pages 695 - 700, XP032196442, ISBN: 978-1-4673-1403-9, DOI: 10.1109/ICRA.2012.6225081
- PHAM LAN N ET AL: "Soft Endoluminal Robots Propelled by Rotating Magnetic Dipole Fields", IEEE TRANSACTIONS ON MEDICAL ROBOTICS AND BIONICS, IEEE, vol. 2, no. 4, 30 September 2020 (2020-09-30), pages 598 - 607, XP011822482, DOI: 10.1109/TMRB.2020.3027871

## Description

### FIELD OF THE INVENTION

This invention relates to magnetic anchored and actuated systems.

### BACKGROUND OF THE INVENTION

Minimally invasive surgery (MIS) has been widely applied in the modern medical field with the clinical benefits of shorter convalescence, less postoperative pain and improved cosmesis. Magnetic anchored and actuated systems have high potential in clinical applications, such as assistive devices for MIS, like the magnetic endoscope systems proposed by Cadeddu *et al.* and Valdastri *et al.* However, the anchoring distance in these systems is short and cannot cover the body cavity thickness. To increase the effective anchoring and actuation distance, larger magnets are required, which complex the system. Magnetic anchored and actuated systems have been developed in research labs aiming for MIS, such as laparoscopic surgery and thoracic surgery. However, none has been adopted clinically. One of the major issues is that the effective magnetic anchoring distance cannot cover the full thickness of the body cavities. Existing methods can provide an anchoring distance that ranges from 20-50 mm, just like the system reported by Liu *et al.* and Cheng *et al,* which uses big magnets to anchor and actuate a capsule-shaped endoscope in the thoracic or abdominal cavity. On the contrary, wall thickness for the chest cavity can reach 80mm or more. Larger magnets can be used to increase the anchoring and actuation distance. However, this will make the system bulky and oversize. The consequence is that the device may not be able to insert into the chest cavity through the intercostal space, or a larger incision port is needed. Other approaches on the problem include the following: XU DONG et al. 2020 IEEE 18TH INTERNATIONAL CONFERENCE ON INDUSTRIAL INFORMATICS (INDIN), IEEE, vol. 1, 20 July 2020 (2020-07-20), pages 225-230; LEONG FLORENCE et al., IEEE REVIEWS IN BIOMEDICAL ENGINEERING, vol. 9, pages 66-78; DINATALI C et al., ROBOTICS AND AUTOMATION (ICRA), 2012 IEEE INTERNATIONAL CONFERENCE ON, IEEE, 14 May 2012, pages 695-700; PHAM LAN N et al., IEEE TRANSACTIONS ON MEDICAL ROBOTICS AND BIONICS, IEEE, vol. 2, no. 4, 30 September 2020 (2020-09-30), pages 598-607; and US 2021/137622 A1.

This invention provides a magnet configuration that could effectively increase the anchoring and actuation distance while keeping the system design compact. Many devices in MIS face the problem of space constraints, such as endoscope, tissue retractor, organ clamp, blood sucker and so on. This configuration can also provide a large working range for surgeons to operate those instruments, making the whole surgery more convenient and reliable. The magnet configuration can realize the benefits of large anchoring distance and compact dimension, making the magnetic actuation instruments can be applied in the actual application in uniport VATS (Video-assisted thoracoscopic surgery). Compared with the traditional MIS devices, they can alleviate the problems of port crowding and instruments fencing for surgeons and relieve the burden of holding and operating these devices by the assistant.

Information that was published before the filing of this disclosure includes those listed under the Reference List.

### SUMMARY OF THE INVENTION

This invention provides a magnetic anchored and actuated system. In one embodiment, said magnetic anchored and actuated system comprises: a) An internal unit for insertion into a patient's body, comprising an internal frame, an internal magnetic rotator, and a function module; and b) An external unit for anchoring and controlling locomotion of said internal unit, comprising an external frame, an external magnetic rotator, and an actuation module; wherein, said internal magnetic rotator comprises an internal shell, a left internal magnetic component, a middle internal magnetic component, and a right internal magnetic component; said external magnetic rotator comprises an external shell, a left external magnetic component, a middle external magnetic component, and a right external magnetic component; said left external magnetic component and said left internal magnetic component are radially magnetized with magnetization direction of said left external magnetic component being same as said left internal magnetic component; said right external magnetic component and said right internal magnetic component are radially magnetized with magnetization direction of said right external magnetic component being same as said right internal magnetic component; each of said left external magnetic component and said left internal magnetic component has a magnetization direction opposite to each of said right external magnetic component and right internal magnetic component; said middle external magnetic component and said middle internal magnetic component are axially magnetized with magnetization direction of said middle external magnetic component being opposite to said middle internal magnetic component; and each of said left internal magnetic component, middle internal magnetic component and right internal magnetic component is magnetically coupled to each of said left external magnetic component, middle external magnetic component, and right external magnetic component respectively; said actuation module controls motion of said external magnetic rotator, said motion comprises rotation and translation; and said function module provides one or more specific functionalities along with movement of said internal magnetic rotator.

A method for assembling the external magnetic rotator of this invention is further provided. In one embodiment, said method comprises the steps of: a) providing a shell body made of non-ferromagnetic materials; b) Placing the middle external magnetic component in middle part of the shell body; c) Placing and fixing a middle plate; d) Inserting two magnetic shielding plates on both sides of the middle external magnetic component to shield magnetic field; e) Installing the left external magnetic component and the right external magnetic component; f) Placing and fixing a right plate and a left plate, wherein said left plate and right plate are made of non-ferromagnetic materials; and g) Remove the two magnetic shielding plates.

### BRIEF DESCRIPTION OF THE FIGURES

**FIG. 1A** and **FIG. 1B** show the overview of the whole magnetic system and simulated clinical application scenario. These include the external holding device **1,** external unit **2** and internal unit **3.** The internal unit is inserted into the patient and anchored on the inner wall of the chest wall **4** thorough the MIS incision **4-1.**
**FIGS. 2A** and **2B** show the structure of the external unit **2** in an embodiment of this invention, which is composed of an external magnetic rotator **2-1,** an external frame **2-2** and an actuation module **2-3.** The actuation module **2-3** is composed of a motor **2-3-1,** an upper pulley **2-3-2,** a synchronous belt **2-3-3** and a lower pulley **2-3-4.**
**FIG. 3A** and **FIG. 3B** show the structure of the external magnet rotator **2-1** in an embodiment of this invention. In this embodiment, the external magnet rotator **2-1** further comprises the LEPM **2-1-1,** the MEPM **2-1-2** and the REPM **2-1-3** that are held by the left plate **2-1-4,** rotator shell **2-1-5,** middle plate **2-1-6** and right plate **2-1-7.**
**FIG. 4A** and **FIG. 4B** show the structure of the external frame **2-2** in an embodiment of this invention. In this embodiment, the external frame **2-2** further comprises upper frame **2-2-1,** the Permalloy plate on the frame **2-2-2,** the lower left frame **2-2-3** and the lower right frame **2-2-4.**
**FIG. 5A** and **FIG. 5B** show the structure of the internal unit **3** in an embodiment of this invention, which is composed of an internal magnetic rotator **3-1,** a function module **3-2** and an internal frame **3-3.** The internal magnet rotator **3-1** further comprises the LIPM **3-1-1,** the MIPM **3-1-2** and the RIPM **3-1-3** that are held by left shell **3-1-4,** the middle shell **3-1-5** and the right shell **3-1-6.** The internal frame **3-3** further comprise the internal left frame **3-3-1** and internal right frame **3-3-2.**
**FIG. 6** shows another approach to place the function module in an embodiment of this invention. In this embodiment, the function module 3-2 is attached on a pneumatic soft bello
**FIG. 7** shows another approach to place the function module in an embodiment of this invention. In this embodiment, the function module **3-2** is attached on a rigid link I **3-1-10,** a joint **3-1-11** and a rigid link II **3-1-12.**
**FIG. 8** shows another approach to place the function module in an embodiment of this invention. In this embodiment, the function module **3-2** is attached on a flexible link **3-1-13.**
**FIG. 9** shows the magnet distribution and configuration of the magnetic system in an embodiment of this invention, the magnets include LEPM **2-1-1,** MEPM **2-1-2,** REPM **2-1-3,** LIPM **3-1-1,** MIPM **3-1-2** and RIPM **3-1-3.**
**FIG. 10A** to **FIG. 10H** show the magnetic field distribution of this invention and another magnet configuration.
**FIG. 11A** and **FIG. 11B** shows the comparison results of the anchoring performance among this invention and some other magnets configuration. (A) is the curve reflecting the relationship between anchoring force (Fz) and distance; (B) is the curve reflecting the relationship between attractive force per unit volume (FPV) and distance.
**FIG. 12A to FIG. 12L** shows the assembly process of the external magnetic rotator in an embodiment of this invention comprising seven steps.

### DETAILED DESCRIPTION OF THE INVENTION

Magnetic forces provide non-contact motion transmission. This enables the development of magnetic anchored and actuated devices that could be placed inside a body cavity and actuated outside the body. With a direct magnetic coupling, the anchoring and effective actuation range is limited due to the exponential attenuation of magnetic field strength. This patent discloses a magnet coupling method that could effectively increase the anchoring and actuation range. With this method, design examples for minimally invasive surgery are also provided.

The magnetic system is composed of two parts, an internal unit that is inside the patient body and an external unit that is outside the patient body. Both the internal unit and the external unit contain magnetic components. The external unit is held and actuated by an external holding device to anchor the internal unit to the patient's inner wall of the body cavity and drive the internal unit to move and rotate with the help of the magnetic coupling.

The external unit is composed of an external magnetic rotator, an external frame and an actuation module. The external magnetic rotator consists of an axially magnetized external permanent magnets (EPM) in the middle part (MEPM) and two radially magnetized EPMs at left and right ends (LEPM and REPM). All the EPMs are fixed on the shell of the external magnetic rotator. The external magnetic rotator is connected to an external frame and can rotate around its central axis with the help of the actuation module. The internal unit is composed of an internal magnetic rotator, a function module and an internal frame. The internal magnetic rotator consists of an axially magnetized internal permanent magnets (IPM) in the middle part (MIPM) and two radially magnetized IPMs at left and right ends (LIPM and RIPM). All the IPMs are fixed on the shell of the internal magnetic rotator. A function module integrating camera, inertial measurement unit (IMU), LEDs and other components can provide the image, orientation feedback, lighting and other information or condition during the surgery. The internal magnetic rotator is connected to an internal frame in some way and can rotate around its central axis.

This invention provides a magnetic anchored and actuated system. In one embodiment, said magnetic anchored and actuated system comprises: a) An internal unit for insertion into a patient's body, comprising an internal frame, an internal magnetic rotator, and a function module; and b) An external unit for anchoring and controlling locomotion of said internal unit, comprising an external frame, an external magnetic rotator, and an actuation module; wherein, said internal magnetic rotator comprises an internal shell, a left internal magnetic component, a middle internal magnetic component, and a right internal magnetic component; said external magnetic rotator comprises an external shell, a left external magnetic component, a middle external magnetic component, and a right external magnetic component; said left external magnetic component and said left internal magnetic component are radially magnetized with magnetization direction of said left external magnetic component being same as said left internal magnetic component; said right external magnetic component and said right internal magnetic component are radially magnetized with magnetization direction of said right external magnetic component being same as said right internal magnetic component; each of said left external magnetic component and said left internal magnetic component has a magnetization direction opposite to each of said right external magnetic component and right internal magnetic component; said middle external magnetic component and said middle internal magnetic component are axially magnetized with magnetization direction of said middle external magnetic component being opposite to said middle internal magnetic component; and each of said left internal magnetic component, middle internal magnetic component and right internal magnetic component is magnetically coupled to each of said left external magnetic component, middle external magnetic component, and right external magnetic component respectively; said actuation module controls motion of said external magnetic rotator, said motion comprises rotation and translation; and said function module provides one or more specific functionalities along with movement of said internal magnetic rotator.

In one embodiment, said left external magnetic component, middle external magnetic component, right external magnetic component, left internal magnetic component, middle internal magnetic component or right internal magnetic component comprises one or more magnets. In another embodiment, said one or more magnets are selected from the group consisting of permanent magnets, electromagnets and soft magnetic materials.

In one embodiment, said left internal magnetic component, right internal magnetic component, left external magnetic component, right external magnetic component, middle internal magnetic component or middle external magnetic component has a shape selected from the group consisting of cylindrical, semi-cylindrical, cuboid, sphere, semi-sphere, and ellipsoid.

In one embodiment, said internal shell holds in place said left internal magnetic component, said middle internal magnetic component and said right internal magnetic component.

In one embodiment, said internal magnetic rotator is connected via a first connection mechanism to said internal frame for rotation around a central axis. In one embodiment, said first connection mechanism comprises one or more selected from the group consisting of bearings, shaft, compliant structure, and soft structure.

In one embodiment, said one or more specific functionalities comprises providing one or more functions selected the group consisting of lighting, imaging, tissue/organ retraction, instrument manipulation, and fluid suction.

In one embodiment, said function module is attached to the internal magnetic rotator by one or more methods selected from the group consisting of glue, movable joints, and compliant/soft structures.

In one embodiment, said external shell comprises a shell body, a left plate, a middle plate and a right plate for holding said left external magnetic component, middle external magnetic component and right external magnetic component respectively; wherein said shell body, left plate and right plate are made of non-ferromagnetic materials.

In one embodiment, said external magnetic rotator further comprises one or more onboard sensors for measuring location and motion. In another embodiment, said one or more onboard sensors are selected from the group consisting of inertial measurement unit, gyroscope, accelerator, hall sensor, encoder, and optical marker.

In one embodiment, said external magnetic rotator is connected via a second connection mechanism to said external frame for rotation around a central axis. In another embodiment, said second connection mechanism comprises one or more selected from the group consisting of bearings, shaft, compliant structure, and soft structure.

In one embodiment, said actuation module comprises an actuator for rotating said external magnetic rotator and an external holding device for spatially moving said external magnetic rotator. In another embodiment, said actuator comprises a motor. In a further embodiment, said external holding device comprises a robot arm.

A method for assembling the external magnetic rotator of this invention is further provided. In one embodiment, said method comprises the steps of: a) providing a shell body made of non-ferromagnetic materials; b) Placing the middle external magnetic component in middle part of the shell body; c) Placing and fixing a middle plate; d) Inserting two magnetic shielding plates on both sides of the middle external magnetic component to shield magnetic field; e) Installing the left external magnetic component and the right external magnetic component; f) Placing and fixing a right plate and a left plate, wherein said left plate and right plate are made of non-ferromagnetic materials; and g) Remove the two magnetic shielding plates.

In one embodiment, said magnetic shielding plates are made of permalloy.

The invention will be better understood by reference to the Experimental Details which follow, but those skilled in the art will readily appreciate that the specific experiments described are only for illustrative purposes and are not meant to limit the invention as described herein, which is defined by the claims that follow thereafter.

It is to be noted that the transitional term "comprising", which is synonymous with "including", "containing" or "characterized by", is inclusive or open-ended and does not exclude additional, un-recited elements or method steps.

### Details of the Invention Operation/Functions

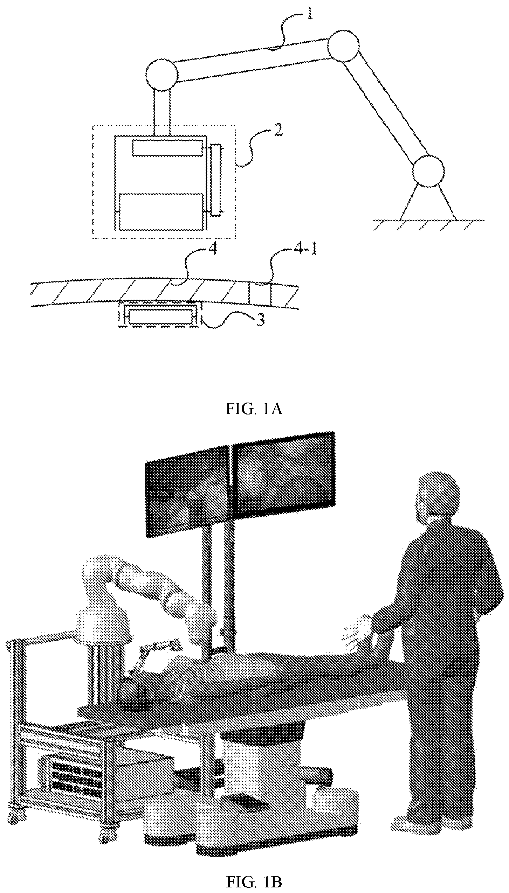

**FIG. 1A** and **FIG. 1B** show the application scenario of this magnetic system in MIS. At the beginning of the surgery, the internal unit **3** will be inserted into the patient's body via the MIS incision **4-1** and be anchored on the patient's inner wall of the cavity by the attractive force generated from the external unit **2.** Next, the external unit **2** held by the external holding device **1** will move to drag the internal unit **3** to the desired position. As the surgical procedure progresses, the external unit **2** can guide the internal unit **3** to translate and rotate to perform corresponding motions to fulfill the surgeon's needs. Finally, the internal unit **3** will move to the vicinity of the MIS incision **4-1** and be taken out.

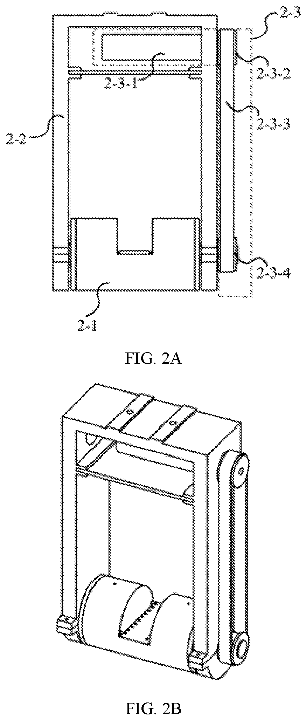

**FIG. 2A** and **FIG. 2B** are an illustration of the external unit **2** which is further composed of the external magnetic rotator **2-1,** an external frame **2-2** and an actuation module **2-3.** In this actuation module **2-3,** the motor **2-3-1** is fixed on the external frame **2-2.** The upper pulley **2-3-2** is fixed on the motor **2-3-1** and connected with the lower pulley **2-3-4** with a synchronous belt **2-3-3.** The lower pulley is fixed on the magnetic rotator **2-1.** So, when the motor **2-3-1** is working, it can actuate the magnetic rotator to rotate.

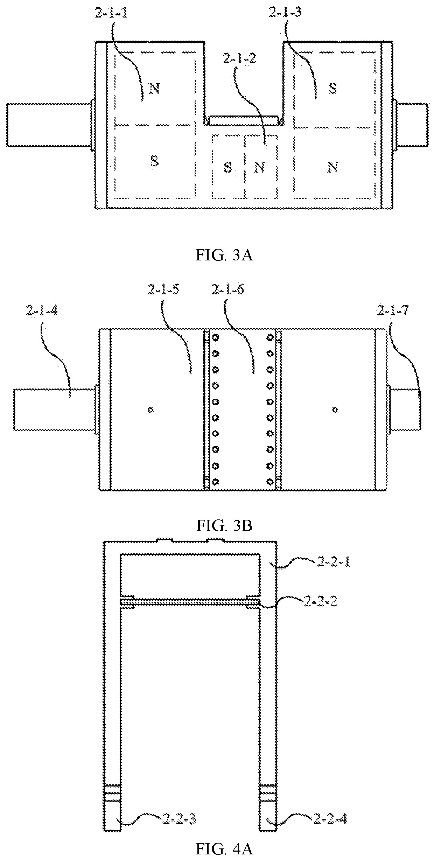

**FIG. 3A** and **FIG. 3B** show the composition of the magnetic rotator **2-1.** It has three magnets. The magnet on the left side (LEPM **2-1-1)** has the magnetization direction from bottom to the top. The magnet on the middle part (MEPM **2-1-2)** has the magnetization direction from left to the right. The magnet on the right side (REPM **2-1-3)** has the magnetization direction from top to the bottom. All the magnets are placed on the rotator shell **2-1-5.** A left plate **2-1-4** is connected to the rotator shell **2-1-5** to hold the LEPM **2-1-1.** A middle plate **2-1-6** is connected to the rotator shell **2-1-5** to hold the MEPM **2-1-2.** A right plate **2-1-7** is connected to the rotator shell **2-1-5** to hold the REPM **2-1-3.**

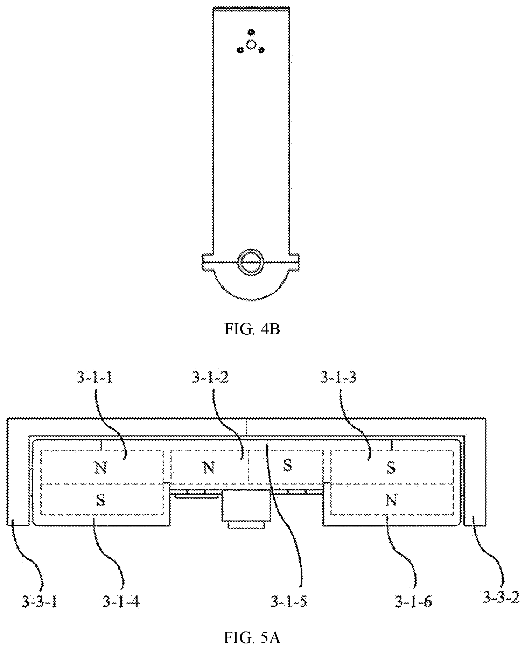

**FIG. 4A** and **FIG. 4B** give one example of the external frame. The upper frame **2-2-1** holds the motor **2-3-1** to actuate the magnetic rotator **2-1** and a Permalloy plate on the frame **2-2-2** to shield the effect of magnetic fields on the motor **2-3-1.** The lower left frame **2-2-3** and the lower right frame **2-2-4** is connected on the upper frame **2-2-1** to hold the magnetic rotator **2-1.**

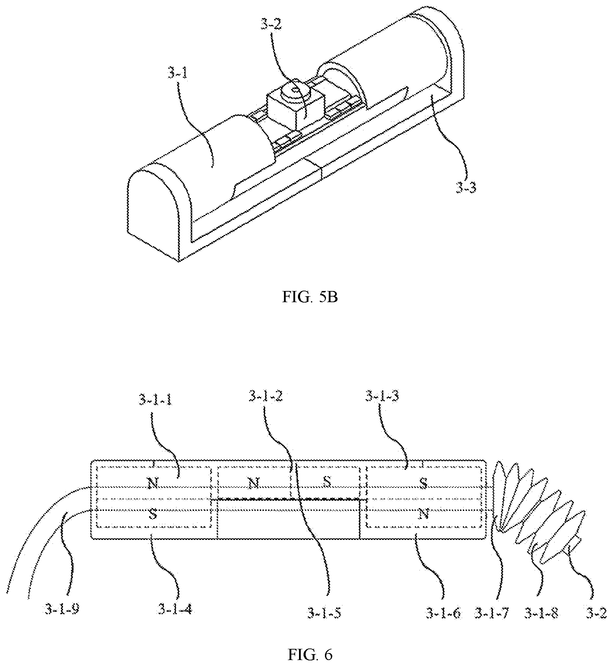

**FIG. 5A** and **FIG. 5B** give one example of the internal unit **3.** It is composed of an internal magnetic rotator **3-1,** a function module **3-2** and an internal frame **3-3.** The internal magnetic rotator **3-1** has three magnets. The magnet on the left side (LIPM **3-1-1)** has the magnetization direction from bottom to the top. The magnet on the middle part (MIPM **3-1-2)** has the magnetization direction from left to the right. The magnet on the right side (REPM **3-1-3)** has the magnetization direction from top to the bottom. A left shell **3-1-4** is used to hold the LIPM **3-1-1.** A middle shell **3-1-5** is used to hold the LIPM **3-1-2.** A right shell **3-1-6** is used to hold the RIPM **3-1-6.** The internal left frame **3-3-1** and internal right frame **3-3-2** are connected to hold the internal magnetic rotator **3-1** that can rotate around its own central axis. The function module **3-2** is placed on the lower half of the middle part. It can provide the image, orientation feedback, lighting and other information or condition during the surgery.

**FIG. 6** gives another example of the internal unit **3.** The magnet on the left side (LIPM **3-1-1)** has the magnetization direction from bottom to the top. The magnet on the middle part (MIPM **3-1-2)** has the magnetization direction from left to the right. The magnet on the right side (REPM **3-1-3)** has the magnetization direction from top to the bottom. A left shell **3-1-4** is used to hold the LIPM **3-1-1.** A middle shell **3-1-5** is used to hold the LIPM **3-1-2.** A right shell **3-1-6** is used to hold the RIPM **3-1-6.** The pneumatic soft bellow I **3-1-7** which can extend the length is attached on the torque coil **3-1-9.** The torque coil **3-1-9** can rotate to provide a rotational degree of freedom. The pneumatic soft bellow II **3-1-8** which can bend is attached on the pneumatic soft bellow I **3-1-7.** And the function module **3-2** is attached on the pneumatic soft bellow II **3-1-8.**

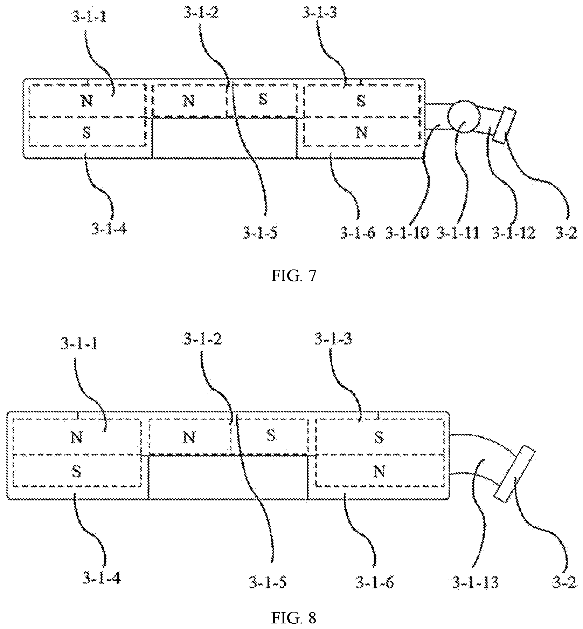

**FIG. 7** gives another example of the internal unit **3.** The magnet on the left side (LIPM **3-1-1)** has the magnetization direction from bottom to the top. The magnet on the middle part (MIPM **3-1-2)** has the magnetization direction from left to the right. The magnet on the right side (REPM **3-1-3)** has the magnetization direction from top to the bottom. A left shell **3-1-4** is used to hold the LIPM **3-1-1.** A middle shell **3-1-5** is used to hold the LIPM **3-1-2.** A right shell **3-1-6** is used to hold the RIPM **3-1-6.** The rigid link I **3-1-10** is attached on the right shell **3-1-6.** The joint **3-1-11** which can be actuated by magnetic field or motor is attached on the rigid link I **3-1-10.** The rigid link II **3-1-12** is attached on the joint **3-1-11.** And the function module **3-2** is attached on the rigid link II **3-1-12.**

**FIG. 8** gives another example of the internal unit **3.** The magnet on the left side (LIPM **3-1-1)** has the magnetization direction from bottom to the top. The magnet on the middle part (MIPM **3-1-2)** has the magnetization direction from left to the right. The magnet on the right side (REPM **3-1-3)** has the magnetization direction from top to the bottom. A left shell **3-1-4** is used to hold the LIPM **3-1-1.** A middle shell **3-1-5** is used to hold the LIPM **3-1-2.** A right shell **3-1-6** is used to hold the RIPM **3-1-6.** The flexible link **3-1-13** is attached on the right shell **3-1-6.** The flexible link **3-1-13** can be soft magnetic material, cable-driven flexible structure and so on. And the function module **3-2** is attached on the flexible link **3-1-13.**

Of course, for the function module, here are many other variations based on this magnet configuration, such as a tissue retractor, organ clamp, blood sucker and so on. They can also be inserted into the patient body and anchored on the inner wall of the body cavity to preform different functions. This design can also enhance their anchoring distance and working stability.

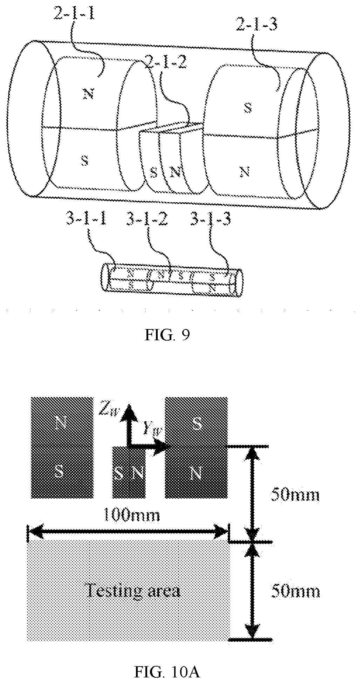

**FIG. 9** show the magnet distribution and configuration of the magnetic system. the magnetization direction of LEPM **2-1-1** and LIPM **2-1-3** is opposite to that of REPM **3-1-1** and RIPM **3-1-3,** leading to the repulsive force between LEPM **2-1-1** and RIPM **3-1-3** and repulsive force between REPM **2-1-3** and LIPM **3-1-1.** This effect will significantly weaken the attractive force applied to the internal unit, resulting in a shorter anchoring distance between the bottom of EPMs and top of IPMs. Therefore, MEPM **2-1-2** and MIPM **3-1-2** are introduced to improve the anchoring performance of the capsule. The magnetic coupling between them can generate extra attractive force to increase the working range. In addition, MEPM **2-1-2** can also attract LIPM **3-1-1** and RIPM **3-1-3,** which is the same as MIPM **3-1-2** can be attracted by LEPM **2-1-1** and REPM **2-1-3,** providing additional anchoring force.

As for the design optimization for this magnet configuration, it is mainly about the size, shape, and strength of the magnets and the distance between the magnets caged in one rotator. These parameters can be determined by the demand of working distance and motion range. The performance of the system can be evaluated by theoretical analysis and finite element analysis. Also, the difficulty of installation, process of fabrication and cost should also be taken into consideration.

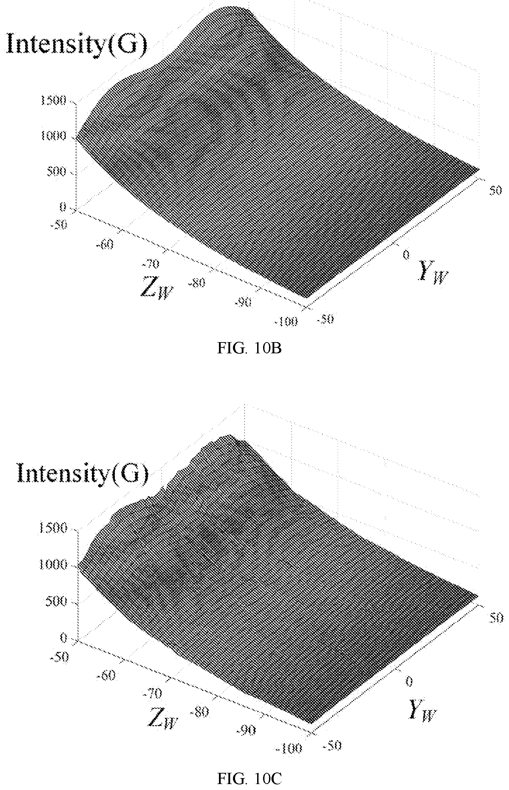

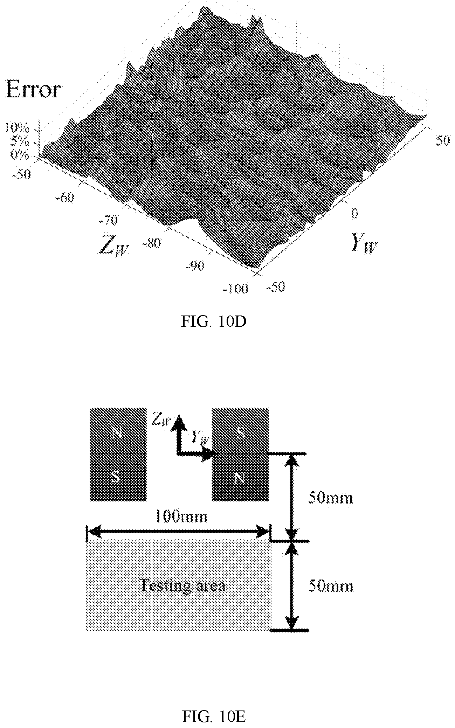

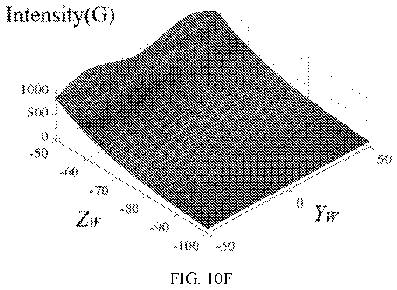

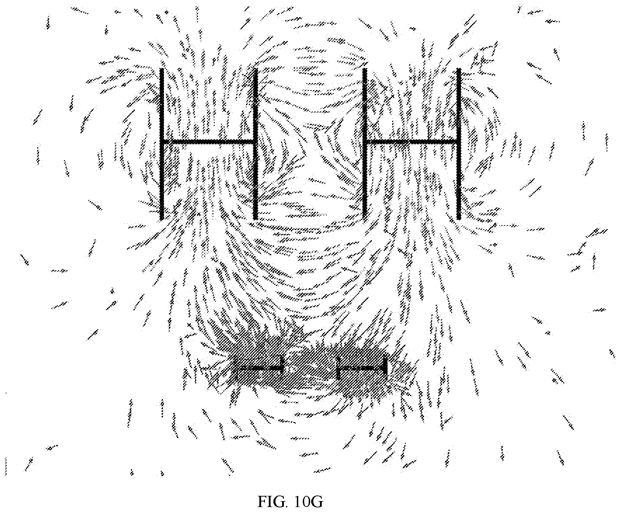

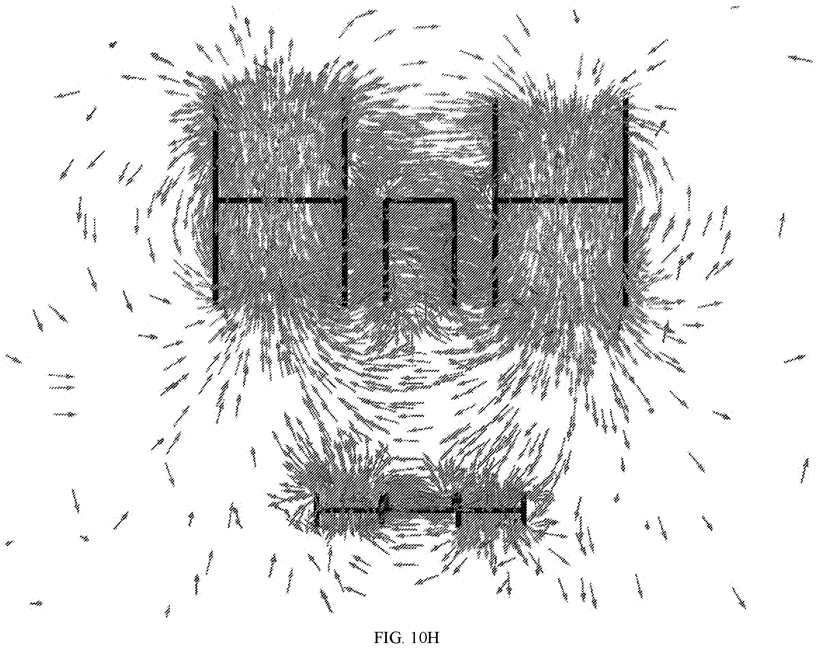

**FIG. 10A** to **FIG. 10H** show the magnetic field distribution of different magnet configurations. In YZ plane, one testing area below the EPMs was selected for evaluation. It had a size of 100 mm×50 mm, and the sampling interval was set as 1 mm (as shown in **FIG. 10A****).** Then, the norm of magnetic field intensity distributed in that region was obtained by model calculation. Here the magnetic dipole model is adopted to get the magnetic field situation, the magnetic field strength generated by the a^{th} EPM at the b^{th} location is obtained by: ${B}_{ab} = \frac{{μ}_{0}}{4 π {\left‖{P}_{lb}-{P}_{Ea}\right‖}^{3}} \left(\frac{3 \left({P}_{lb}-{P}_{Ea}\right) {\left({P}_{lb}-{P}_{Ea}\right)}^{T}}{{\left‖{P}_{lb}-{P}_{Ea}\right‖}^{2}}-I\right) {R}_{E} {M}_{Ea}$ where *µ₀* is the vacuum permeability that is a constant; $I ∈ {ℝ}^{3 \times 3}$ is an identity matrix; ${M}_{Ea} = {\left[{m}_{Eax}, {m}_{Eay}, {m}_{Eaz}\right]}^{T} ∈ {ℝ}^{3 \times 1}$ indicates the magnetic moment vector of the a^{th} EPM at initial position; ${R}_{E} ∈ {ℝ}^{3 \times 3}$ is the rotation matrix of the external magnetic rotator. Then superpose the magnetic field of all the EPMs and calculate the norm of the magnetic field to get the result of the **FIG. 10B****.** The finite element analysis is also performed to verify the theoretical model. The result is shown in **FIG. 10C** and the error situation is shown in **FIG. 10D****.** It can be seen that the result of model of and simulation can math very well. Another magnet configuration is picked to compare the magnetic field distribution that is shown in **FIG. 10E** and **FIG. 10F****.** And the overall magnetic field distribution for two designs is shown in **FIG. 10G** and **FIG. 10H****.** It can be seen that the magnet configuration in this invention can strengthen the magnetic field significantly over the other existing magnet configuration.

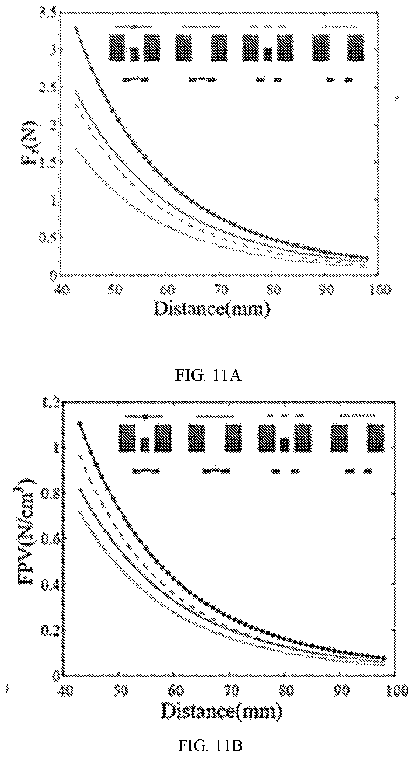

In **FIG. 11A** and **FIG. 11B****,** we select serval magnet configurations, the remanence of EPMs and IPMs are all set the same. The curve reflecting the relationship between the anchoring force and distance is obtained from the magnetic model calculation after the experimental verification. The resultant force acting on the IPMs immersed in the magnetic field is calculated by: ${F}_{C} = {\sum }_{a = 1}^{n} {\sum }_{b = 1}^{n} \left({R}_{I}{M}_{Ib}⋅∇\right) {B}_{ab}$ where
${M}_{Ib} = {\left[{m}_{Ibx}, {m}_{Iby}, {m}_{Ibz}\right]}^{T} ∈ {ℝ}^{3 \times 1}$ represents the magnetic moment of the b^{th} IPM at the initial position. ${R}_{I} ∈ {ℝ}^{3 \times 3}$ is the rotation matrix of the internal magnetic rotator. Every force can be further transformed as: ${F}_{ab} = \frac{3 {μ}_{0}}{4 π {\left‖{r}_{ab}\right‖}^{4}} \left({R}_{I}{M}_{Ib}{\hat{r}}_{ab}^{T}+{\hat{r}}_{ab}{\left({R}_{I}{M}_{Ib}\right)}^{T}+\left({\hat{r}}_{ab}⋅\left({R}_{I}{M}_{Ib}\right)\right)\left(I-5{\hat{r}}_{ab}{\hat{r}}_{ab}^{T}\right)\right) {R}_{E} {M}_{EA}$ Where *r_{ab} = P_{b} - Pₐ* is used to denote the space the vector from the a^{th} external magnetic dipole to b^{th} internal magnetic dipole. *r̂_{ab}* represents the normalized vector of *r_{ab}.* **FIG. 11A** gives the result of the anchoring force of different magnet configuration.

Of course, the volume of magnets in different configurations is not all the same, so the unified evaluation index called attractive force per unit volume (FPV) is brought up: $FPV = \frac{{F}_{z}}{{V}_{IPMs}}$

The index includes two most important aspects for clinical application in VATS: a compact dimension of the internal unit that can be inserted through the narrow incision and considerable anchoring force to hold the endoscope on the thick chest wall. The result of this index is shown in **FIG. 11B****.** The comparison results manifest the magnet configuration proposed in this paper can significantly enhance the anchoring performance, the increase of attractive force and FPV is 96.76% and 55.82% averagely.

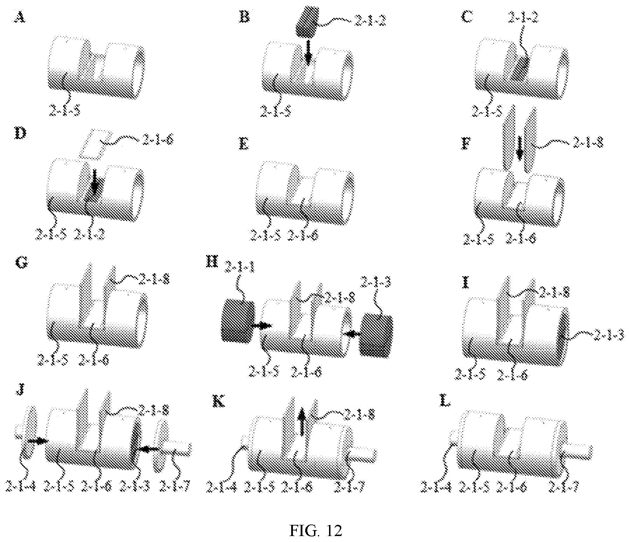

**FIG. 12** shows the installation process. During this process, there is a need to reduce the huge repulsive force between the magnets. This invention provides an installation method to overcome this problem:
Step1. Prepare the rotator shell **2-1-5.**
Step2. Place the MEPM **2-1-2** in the middle part of the rotator shell **2-1-5.**
Step3. Place the middle plate **2-1-6** and fix it on the rotator shell **2-1-5.**
Step4. Insert two Permalloy plates for the rotator **2-1-8** on both sides of the MEPM **2-1-2** to shield magnetic field.
Step5. Install the LEPM **2-1-1** and REPM **2-1-3.**
Step6. Place the left plate **2-1-4** and right plate **2-1-7** and fix them on the rotator shell **2-1-5.**
Step7. Remove the two Permalloy plates for the rotator **2-1-8.**

As for the working principle of the system, utilizing the magnetic coupling between the EPMs and IPMs, the configuration can realize the rotation motion and translation motion under the premise of anchoring the capsule against the inner wall of the body cavity. The rotation of the internal unit includes the tilt motion and pan motion, and are achieved by the torques applied by EPMs around the corresponding axis. The tilt torque and pan torque acting on the capsule is generated by rotating the external unit along the corresponding axis to change the EPMs' magnetic field coupled to the IPMs. The translation motion of the internal endoscope is on the curved surface of the chest wall, requiring the magnetic force along the corresponding axis. The movement of the external unit above the patient's body cavity can produce the translational force.

### Manufacturing Method

For the magnets, they can be permanent magnet, electromagnet, magnetized soft materials and so on. They can be made in the lab, bought from the store or produced by factory. For the shell, plate, and frame, they can be fabricated with the material with high strength and connected with the method with high stability.

### Reference List

1. U.S. Pat. No. 6,902,528 B1. Method and apparatus for magnetically controlling catheters in body lumens and cavities, 18 Feb. 2003.
2. J. Cadeddu, R. Fernandez, M. Desai, R. Bergs, C. Tracy, S.-J. Tang, P. Rao, M. Desai, and D. Scott, "Novel magnetically guided intraabdominal camera to facilitate laparoendoscopic single-site surgery: initial human experience," Surgical endoscopy, vol. 23, no. 8, pp. 1894-1899, 2009.
3. M. Simi, M. Silvestri, C. Cavallotti, M. Vatteroni, P. Valdastri, A. Menciassi, and P. Dario, "Magnetically activated stereoscopic vision system for laparoendoscopic single-site surgery," IEEE/ASME Transactions on Mechatronics, vol. 18, no. 3, pp. 1140-1151, 2012.
4. M. Simi, R. Pickens, A. Menciassi, S. D. Herrell, and P. Valdastri, "Fine tilt tuning of a laparoscopic camera by local magnetic actuation: Two-port nephrectomy experience on human cadavers," Surgical innovation, vol. 20, no. 4, pp. 385-394, 2013.
5. N. Garbin, P. R. Slawinski, G. Aiello, C. Karraz, and P. Valdastri, "Laparoscopic camera based on an orthogonal magnet arrangement," IEEE Robotics and Automation Letters, vol. 1, no. 2, pp. 924-929, 2016.
6. X. Liu, G. J. Mancini, and J. Tan, "Design of a unified active locomotion mechanism for a wireless laparoscopic camera system," in 2014 IEEE/RSJ International Conference on Intelligent Robots and Systems. IEEE, 2014, pp. 1294-1301
7. X. Liu, G. J. Mancini, and J. Tan, "Design of a unified active locomotion mechanism for a capsule-shaped laparoscopic camera system," in 2014 IEEE International Conference on Robotics and Automation (ICRA). IEEE, 2014, pp. 2449-2456.
8. H. Wei, W. Tan, K. Li, and D. Xu, "Design and modeling of a magnetically actuated laparoscopic robot," in 2019 14th IEEE Conference on Industrial Electronics and Applications (ICIEA). IEEE, 2019, pp. 149-154.
9. T. Cheng, C. S. H. Ng, P. W. Y. Chiu, and Z. Li, "Design and prototyping of a soft magnetic anchored and guidance endoscope system," in 2017 IEEE/RSJ International Conference on Intelligent Robots and Systems (IROS). IEEE, 2017, pp. 2902-2908.
10. T. Cheng, X. Zhang, C. S. H. Ng, P. W. Y. Chiu, and Z. Li, "A novel magnetic anchored and steered camera robot for single port access surgery," in 2018 IEEE International Conference on Robotics and Automation (ICRA). IEEE, 2018, pp. 1406-1412.
11. X. Liu, G. J. Mancini, Y. Guan, and J. Tan, "Design of a magnetic actuated fully insertable robotic camera system for single-incision laparoscopic surgery," IEEE/ASME Transactions on Mechatronics, vol. 21, no. 4, pp. 1966-1976, 2015.
12. T. Cheng, W. Li, C. S. H. Ng, P. W. Y. Chiu, and Z. Li, "Visual servo control of a novel magnetic actuated endoscope for uniportal videoassisted thoracic surgery," IEEE Robotics and Automation Letters, vol. 4, no. 3, pp. 3098-3105, 2019.
13. T. Cheng, W. Li, W. Y. Ng, Y. Huang, J. Li, C. S. H. Ng, P. W. Y. Chiu, and Z. Li, "Deep learning assisted robotic magnetic anchored and guided endoscope for real-time instrument tracking," IEEE Robotics and Automation Letters, vol. 6, no. 2, pp. 3979-3986, 2021.
14. U.S. Pat. No. 8,084,898 B2. Magnetic actuator, magnetic actuator operating method, and capsule endoscope using the same, 27 Dec. 2011.
15. U.S. Pat. No. 11,033,183 B2. Wireless magnetically steerable endoscope, 15 Jun. 2021.

## Claims

1. A magnetic anchored and actuated system, comprising:
a. An internal unit (3) for insertion into a patient's body, comprising an internal frame (3-3), an internal magnetic rotator (3-1), and a function module (3-2); and
b. An external unit (2) for anchoring and controlling locomotion of said internal unit (3), comprising an external frame (2-2), an external magnetic rotator (2-1), and an actuation module (2-3);
wherein,
said internal magnetic rotator (3-1) comprises an internal shell, a left internal magnetic component (3-1-1), a middle internal magnetic component (3-1-2), and a right internal magnetic component (3-1-3);
said external magnetic rotator (2-1) comprises an external shell (2-1-5), a left external magnetic component (2-1-1), a middle external magnetic component (2-1-2), and a right external magnetic component (2-1-3);
said left external magnetic component (2-1-1) and said left internal magnetic component (3-1-1) are radially magnetized with magnetization direction of said left external magnetic component being same as said left internal magnetic component;
said right external magnetic component (2-1-3) and said right internal magnetic component (3-1-3) are radially magnetized with magnetization direction of said right external magnetic component (2-1-3) being same as said right internal magnetic component (3-1-3);
each of said left external magnetic component (2-1-1) and said left internal magnetic component (3-1-1) has a magnetization direction opposite to each of said right external magnetic component (2-1-3) and right internal magnetic component (3-1-3);
and
each of said left internal magnetic component (3-1-1), middle internal magnetic component (3-1-2) and right internal magnetic component (3-1-3) is magnetically coupled to each of said left external magnetic component (2-1-1), middle external magnetic component (2-1-2), and right external magnetic component (2-1-3) respectively;
said actuation module (2-3) controls motion of said external magnetic rotator (2-1), said motion comprises rotation and translation; and
said function module (3-2) provides one or more specific functionalities along with movement of said internal magnetic rotator (3-1);
**characterized in that**:
said middle external magnetic component (2-1-2) and said middle internal magnetic component (3-1-2) are axially magnetized with magnetization direction of said middle external magnetic component (2-1-2) being opposite to said middle internal magnetic component (3-1-2).

2. The system of claim 1, wherein each one of said left external magnetic component (2-1-1), middle external magnetic component (2-1-2), right external magnetic component (2-1-3), left internal magnetic component (3-1-1), middle internal magnetic component (3-1-2) or right internal magnetic component (3-1-3) comprises one or more magnets.

3. The system of claim 2, wherein said one or more magnets are selected from the group consisting of permanent magnets, electromagnets and soft magnetic materials.

4. The system of claim 1, wherein said left internal magnetic component (3-1-1), right internal magnetic component (3-1-3), left external magnetic component (2-1-1), right external magnetic component (2-1-3), middle internal magnetic component (3-1-2) or middle external magnetic component (2-1-2) has a shape selected from the group consisting of cylindrical, semi-cylindrical, cuboid, sphere, semi-sphere, and ellipsoid.

5. The system of claim 1, wherein said internal shell holds in place said left internal magnetic component (3-1-1), said middle internal magnetic component (3-1-2) and said right internal magnetic component (3-1-3).

6. The system of claim 1, wherein said internal magnetic rotator (3-1) further comprises one or more onboard sensors for measuring location and motion.

7. The system of claim 6, wherein said one or more onboard sensors are selected from the group consisting of inertial measurement unit, gyroscope, accelerator, hall sensor, and optical marker.

8. The system of claim 1, wherein said internal magnetic rotator (3-1) is connected via a first connection mechanism to said internal frame (3-3) for rotation around a central axis.

9. The system of claim 8, wherein said first connection mechanism comprises one or more selected from the group consisting of bearings, shaft, compliant structure, and soft structure.

10. The system of claim 1, wherein said one or more specific functionalities comprises providing one or more functions selected the group consisting of lighting, imaging, tissue/organ retraction, instrument manipulation, and fluid suction.

11. The system of claim 1, wherein said function module (3-2) is attached to the internal magnetic rotator (3-1) by one or more methods selected from the group consisting of glue, movable joints, and compliant/soft structures.

12. The system of claim 1, wherein said external shell comprises a shell body (2-1-5), a left plate (2-1-4), a middle plate (2-1-6) and a right plate (2-1-7) for holding said left external magnetic component (2-1-1), middle external magnetic component (2-1-2) and right external magnetic component (2-1-3) respectively; wherein said shell body (2-1-5), left plate (2-1-4) and right plate (2-1-7) are made of non-ferromagnetic materials.

13. The system of claim 1, wherein said external magnetic rotator (2-1) further comprises one or more onboard sensors for measuring location and motion.

14. The system of claim 13, wherein said one or more onboard sensors are selected from the group consisting of inertial measurement unit, gyroscope, accelerator, hall sensor, encoder, and optical marker.

15. The system of claim 8, wherein said external magnetic rotator (2-1) is connected via a second connection mechanism to said external frame (2-2) for rotation around a central axis.

16. The system of claim 15, wherein said second connection mechanism comprises one or more selected from the group consisting of bearings, shaft, compliant structure, and soft structure.

17. The system of claim 1, wherein said actuation module (2-3) comprises an actuator for rotating said external magnetic rotator (2-1) and an external holding device for spatially moving said external magnetic rotator (2-1).

18. The system of claim 17, wherein said actuator comprises a motor.

19. The system of claim 17, wherein said external holding device (1) comprises a robot arm.

20. A method for assembling the external magnetic rotator in the system of claim 1, said method comprises the steps of:
a. providing a shell body made of non-ferromagnetic materials;
b. Placing the middle external magnetic component in middle part of said shell body;
c. Placing and fixing a middle plate;
d. Inserting two magnetic shielding plates on both sides of the middle external magnetic component to shield magnetic field;
e. Installing the left external magnetic component and the right external magnetic component;
f. Placing and fixing a right plate and a left plate, wherein said left plate and right plate are made of non-ferromagnetic materials; and
g. Remove the two magnetic shielding plates.

21. The method of claim 20, wherein said magnetic shielding plates are made of permalloy.

## Patentansprüche

1. Magnetisches verankertes und betätigtes System, umfassend:
a. eine innere Einheit (3) zum Einsetzen in einen Körper eines Patienten, die einen inneren Rahmen (3-3), einen inneren magnetischen Rotator (3-1) und ein Funktionsmodul (3-2) umfasst; und
b. eine äußere Einheit (2) zum Verankern und Steuern von Fortbewegung der inneren Einheit (3), umfassend einen äußeren Rahmen (2-2), einen äußeren magnetischen Rotator (2-1) und ein Betätigungsmodul (2-3);
wobei,
der innere magnetische Rotator (3-1) eine innere Schale, eine linke innere magnetische Komponente (3-1-1), eine mittlere innere magnetische Komponente (3-1-2) und eine rechte innere magnetische Komponente (3-1-3) umfasst;
der äußere magnetische Rotator (2-1) eine äußere Schale (2-1-5), eine linke äußere magnetische Komponente (2-1-1), eine mittlere äußere magnetische Komponente (2-1-2) und eine rechte äußere magnetische Komponente (2-1-3) umfasst;
die linke äußere magnetische Komponente (2-1-1) und die linke innere magnetische Komponente (3-1-1) radial magnetisiert sind, wobei die Magnetisierungsrichtung der linken äußeren magnetischen Komponente die gleiche ist wie die der linken inneren magnetischen Komponente;
die rechte äußere magnetische Komponente (2-1-3) und die rechte innere magnetische Komponente (3-1-3) radial magnetisiert sind, wobei die Magnetisierungsrichtung der rechten äußeren magnetischen Komponente (2-1-3) die gleiche ist wie die der rechten inneren magnetischen Komponente (3-1-3);
jede der linken äußeren magnetischen Komponente (2-1-1) und der linken inneren magnetischen Komponente (3-1-1) eine zu der rechten äußeren magnetischen Komponente (2-1-3) und rechten inneren magnetischen Komponente (3-1-3) entgegengesetzte Magnetisierungsrichtung aufweist;
und
jede der linken inneren magnetischen Komponente (3-1-1), mittleren inneren magnetischen Komponente (3-1-2) und rechten inneren magnetischen Komponente (3-1-3) mit jeder der linken äußeren magnetischen Komponente (2-1-1), mittleren äußeren magnetischen Komponente (2-1-2) und rechten äußeren magnetischen Komponente (2-1-3) jeweils magnetisch gekoppelt ist;
das Betätigungsmodul (2-3) die Bewegung des äußeren magnetischen Rotators (2-1) steuert, wobei die Bewegung eine Rotation und Translation umfasst; und
das Funktionsmodul (3-2) eine oder mehrere spezifische Funktionalitäten zusammen mit der Bewegung des inneren magnetischen Rotators (3-1) bereitstellt;
**dadurch gekennzeichnet, dass**:
die mittlere äußere magnetische Komponente (2-1-2) und die mittlere innere magnetische Komponente (3-1-2) axial magnetisiert sind, wobei die Magnetisierungsrichtung der mittleren äußeren magnetischen Komponente (2-1-2) entgegengesetzt zu der mittleren inneren magnetischen Komponente (3-1-2) ist.

2. System nach Anspruch 1, wobei jede einzelne der linken äußeren magnetischen Komponente (2-1-1), mittleren äußeren magnetischen Komponente (2-1-2), rechten äußeren magnetischen Komponente (2-1-3), linken inneren magnetischen Komponente (3-1-1), mittleren inneren magnetischen Komponente (3-1-2) oder rechten inneren magnetischen Komponente (3-1-3) einen oder mehrere Magneten umfasst.

3. System nach Anspruch 2, wobei der eine oder die mehreren Magnete ausgewählt sind aus der Gruppe bestehend aus Permanentmagneten, Elektromagneten und weichmagnetischen Materialien.

4. System nach Anspruch 1, wobei die linke innere magnetische Komponente (3-1-1), rechte innere magnetische Komponente (3-1-3), linke äußere magnetische Komponente (2-1-1), rechte äußere magnetische Komponente (2-1-3), mittlere innere magnetische Komponente (3-1-2) oder mittlere äußere magnetische Komponente (2-1-2) eine Form aufweist, die ausgewählt ist aus der Gruppe bestehend aus zylindrisch, halbzylindrisch, quaderförmig, kugelförmig, halbkugelförmig und ellipsoid.

5. System nach Anspruch 1, wobei die innere Schale die linke innere magnetische Komponente (3-1-1), die mittlere innere magnetische Komponente (3-1-2) und die rechte innere magnetische Komponente (3-1-3) in Position hält.

6. System nach Anspruch 1, wobei der innere magnetische Rotator (3-1) weiter einen oder mehrere integrierte Sensoren zum Messen von Position und Bewegung umfasst.

7. System nach Anspruch 6, wobei der eine oder die mehreren integrierten Sensoren ausgewählt sind aus der Gruppe bestehend aus Trägheitsmesseinheit, Gyroskop, Beschleuniger, Hallsensor und optischem Marker.

8. System nach Anspruch 1, wobei der innere magnetische Rotator (3-1) über einen ersten Verbindungsmechanismus mit dem inneren Rahmen (3-3) zum Rotieren um eine zentrale Achse verbunden ist.

9. System nach Anspruch 8, wobei der erste Verbindungsmechanismus eines oder mehreres umfasst, ausgewählt aus der Gruppe bestehend aus Lagern, Welle, nachgiebiger Struktur und weicher Struktur.

10. System nach Anspruch 1, wobei die eine oder die mehreren spezifischen Funktionalitäten Bereitstellen von einer oder mehreren Funktionen umfassen, ausgewählt aus der Gruppe bestehend aus Beleuchtung, Bildgebung, Gewebe-/ Organretraktion, Instrumentenmanipulation und Fluidansaugung.

11. System nach Anspruch 1, wobei das Funktionsmodul (3-2) an dem inneren magnetischen Rotator (3-1) durch ein oder mehrere Verfahren befestigt ist, ausgewählt aus der Gruppe bestehend aus Klebstoff, beweglichen Gelenken und nachgiebigen/weichen Strukturen.

12. System nach Anspruch 1, wobei die äußere Schale einen Schalenkörper (2-1-5), eine linke Platte (2-1-4), eine mittlere Platte (2-1-6) und eine rechte Platte (2-1-7) zum Halten jeweils der linken äußeren magnetischen Komponente (2-1-1), mittleren äußeren magnetischen Komponente (2-1-2) und rechten äußeren magnetischen Komponente (2-1-3) umfasst; wobei der Schalenkörper (2-1-5), die linke Platte (2-1-4) und die rechte Platte (2-1-7) aus nicht ferromagnetischen Materialien hergestellt sind.

13. System nach Anspruch 1, wobei der äußere magnetische Rotator (2-1) weiter einen oder mehrere integrierte Sensoren zum Messen von Position und Bewegung umfasst.

14. System nach Anspruch 13, wobei der eine oder die mehreren integrierten Sensoren ausgewählt sind aus der Gruppe bestehend aus Trägheitsmesseinheit, Gyroskop, Beschleuniger, Hallsensor, Encoder und optischem Marker.

15. System nach Anspruch 8, wobei der äußere magnetische Rotator (2-1) über einen zweiten Verbindungsmechanismus mit dem äußeren Rahmen (2-2) zum Rotieren um eine zentrale Achse verbunden ist.

16. System nach Anspruch 15, wobei der zweite Verbindungsmechanismus eines oder mehreres umfasst, ausgewählt aus der Gruppe bestehend aus Lagern, Welle, nachgiebiger Struktur und weicher Struktur.

17. System nach Anspruch 1, wobei das Betätigungsmodul (2-3) eine Betätigungsvorrichtung zum Rotieren des äußeren magnetischen Rotators (2-1) und eine äußere Haltevorrichtung zum räumlichen Bewegen des äußeren magnetischen Rotators (2-1) umfasst.

18. System nach Anspruch 17, wobei die Betätigungsvorrichtung einen Motor umfasst.

19. System nach Anspruch 17, wobei die äußere Haltevorrichtung (1) einen Roboterarm umfasst.

20. Verfahren zum Montieren des äußeren magnetischen Rotators in das System nach Anspruch 1, wobei das Verfahren die folgenden Schritte umfasst:
a. Bereitstellen eines Schalenkörpers, der aus nicht ferromagnetischen Materialien hergestellt ist;
b. Platzieren der mittleren äußeren magnetischen Komponente im mittleren Teil des Schalenkörpers;
c. Platzieren und Fixieren einer mittleren Platte;
d. Einsetzen von zwei magnetischen Abschirmungsplatten an beiden Seiten der mittleren äußeren magnetischen Komponente, um Magnetfeld abzuschirmen;
e. Montieren der linken äußeren magnetischen Komponente und der rechten äußeren magnetischen Komponente;
f. Platzieren und Fixieren einer rechten Platte und einer linken Platte, wobei die linke Platte und die rechte Platte aus nicht ferromagnetischen Materialien hergestellt sind; und
g. Entfernen der zwei magnetischen Abschirmungsplatten.

21. Verfahren nach Anspruch 20, wobei die magnetischen Abschirmungsplatten aus Permalloy hergestellt sind.

## Revendications

1. Système magnétique ancré et actionné, comprenant :
a. une unité interne (3) destinée à être insérée dans le corps d'un patient, comprenant un cadre interne (3-3), un rotateur magnétique interne (3-1) et un module de fonction (3-2) ; et
b. une unité externe (2) destinée à ancrer et à commander le déplacement de ladite unité interne (3), comprenant un cadre externe (2-2), un rotateur magnétique externe (2-1) et un module d'actionnement (2-3) ;
dans lequel,
ledit rotateur magnétique interne (3-1) comprend une coque interne, un composant magnétique interne gauche (3-1-1), un composant magnétique interne médian (3-1-2) et un composant magnétique interne droit (3-1-3) ;
ledit rotateur magnétique externe (2-1) comprend une coque externe (2-1-5), un composant magnétique externe gauche (2-1-1), un composant magnétique externe médian (2-1-2) et un composant magnétique externe droit (2-1-3) ;
ledit composant magnétique externe gauche (2-1-1) et ledit composant magnétique interne gauche (3-1-1) sont magnétisés radialement, la direction de magnétisation dudit composant magnétique externe gauche étant la même que celle dudit composant magnétique interne gauche ;
ledit composant magnétique externe droit (2-1-3) et ledit composant magnétique interne droit (3-1-3) sont magnétisés radialement, la direction de magnétisation dudit composant magnétique externe droit (2-1-3) étant la même que celle dudit composant magnétique interne droit (3-1-3) ;
ledit composant magnétique externe gauche (2-1-1) et ledit composant magnétique interne gauche (3-1-1) présentent chacun une direction de magnétisation opposée à chacun dudit composant magnétique externe droit (2-1-3) et dudit composant magnétique interne droit (3-1-3) ;
et
ledit composant magnétique interne gauche (3-1-1), ledit composant magnétique interne médian (3-1-2) et ledit composant magnétique interne droit (3-1-3) sont chacun respectivement couplés magnétiquement à chacun dudit composant magnétique externe gauche (2-1-1), dudit composant magnétique externe médian (2-1-2) et dudit composant magnétique externe droit (2-1-3) ;
ledit module d'actionnement (2-3) commande le mouvement dudit rotateur magnétique externe (2-1), ledit mouvement comprend une rotation et une translation ; et
ledit module de fonction (3-2) procure une ou plusieurs fonctionnalités spécifiques conjointement avec le mouvement dudit rotateur magnétique interne (3-1) ;
**caractérisé en ce que** :
ledit composant magnétique externe médian (2-1-2) et ledit composant magnétique interne médian (3-1-2) sont magnétisés axialement, la direction de magnétisation dudit composant magnétique externe médian (2-1-2) étant opposée à celle dudit composant magnétique interne médian (3-1-2).

2. Système selon la revendication 1, dans lequel ledit composant magnétique externe gauche (2-1-1), ledit composant magnétique externe médian (2-1-2), ledit composant magnétique externe droit (2-1-3), ledit composant magnétique interne gauche (3-1-1), ledit composant magnétique interne médian (3-1-2) ou ledit composant magnétique interne droit (3-1-3) comprennent chacun un ou plusieurs aimants.

3. Système selon la revendication 2, dans lequel lesdits un ou plusieurs aimants sont sélectionnés dans le groupe consistant en aimants permanents, électroaimants et matériaux magnétiques doux.

4. Système selon la revendication 1, dans lequel ledit composant magnétique interne gauche (3-1-1), composant magnétique interne droit (3-1-3), composant magnétique externe gauche (2-1-1), composant magnétique externe droit (2-1-3), composant magnétique interne médian (3-1-2) ou composant magnétique externe médian (2-1-2) présente une forme sélectionnée dans le groupe consistant en cylindrique, semi-cylindrique, cuboïde, sphère, semi-sphère et ellipsoïde.

5. Système selon la revendication 1, dans lequel ladite coque interne maintient en place ledit composant magnétique interne gauche (3-1-1), ledit composant magnétique interne médian (3-1-2) et ledit composant magnétique interne droit (3-1-3).

6. Système selon la revendication 1, dans lequel ledit rotateur magnétique interne (3-1) comprend en outre un ou plusieurs capteurs embarqués destinés à mesurer l'emplacement et le mouvement.

7. Système selon la revendication 6, dans lequel lesdits un ou plusieurs capteurs embarqués sont sélectionnés dans le groupe consistant en unité de mesure inertielle, gyroscope, accéléromètre, capteur Hall et marqueur optique.

8. Système selon la revendication 1, dans lequel ledit rotateur magnétique interne (3-1) est relié par l'intermédiaire d'un premier mécanisme de liaison audit cadre interne (3-3) pour une rotation autour d'un axe central.

9. Système selon la revendication 8, dans lequel ledit premier mécanisme de liaison comprend un ou plusieurs éléments sélectionnés dans le groupe consistant en roulements, arbre, structure déformable et structure souple.

10. Système selon la revendication 1, dans lequel lesdites une ou plusieurs fonctionnalités spécifiques comprennent la fourniture d'une ou de plusieurs fonctions sélectionnées dans le groupe consistant en éclairage, imagerie, rétraction de tissu/d'organe, manipulation d'instrument et aspiration de fluide.

11. Système selon la revendication 1, dans lequel ledit module de fonction (3-2) est fixé au rotateur magnétique interne (3-1) par un ou plusieurs procédés sélectionnés dans le groupe consistant en colle, raccords mobiles et structures déformables/souples.

12. Système selon la revendication 1, dans lequel ladite coque externe comprend un corps (2-1-5) de coque, une plaque gauche (2-1-4), une plaque médiane (2-1-6) et une plaque droite (2-1-7) pour maintenir en place respectivement lesdits composant magnétique externe gauche (2-1-1), composant magnétique externe médian (2-1-2) et composant magnétique externe droit (2-1-3) ; dans lequel ledit corps (2-1-5) de coque, la plaque gauche (2-1-4) et la plaque droite (2-1-7) sont réalisés en matériaux non ferromagnétiques.

13. Système selon la revendication 1, dans lequel ledit rotateur magnétique externe (2-1) comprend en outre un ou plusieurs capteurs embarqués destinés à mesurer l'emplacement et le mouvement.

14. Système selon la revendication 13, dans lequel lesdits un ou plusieurs capteurs embarqués sont sélectionnés dans le groupe consistant en unité de mesure inertielle, gyroscope, accéléromètre, capteur Hall, codeur et marqueur optique.

15. Système selon la revendication 8, dans lequel ledit rotateur magnétique externe (2-1) est relié par l'intermédiaire d'un deuxième mécanisme de liaison audit cadre externe (2-2) pour une rotation autour d'un axe central.

16. Système selon la revendication 15, dans lequel ledit deuxième mécanisme de liaison comprend un ou plusieurs éléments sélectionnés dans le groupe consistant en roulements, arbre, structure déformable et structure souple.

17. Système selon la revendication 1, dans lequel ledit module d'actionnement (2-3) comprend un actionneur destiné à faire tourner ledit rotateur magnétique externe (2-1) et un dispositif de maintien externe destiné à déplacer spatialement ledit rotateur magnétique externe (2-1).

18. Système selon la revendication 17, dans lequel ledit actionneur comprend un moteur.

19. Système selon la revendication 17, dans lequel ledit dispositif de maintien externe (1) comprend un bras de robot.

20. Procédé destiné à assembler le rotateur magnétique externe dans le système selon la revendication 1, ledit procédé comprend les étapes consistant à :
a. fournir un corps de coque réalisé en matériaux non ferromagnétiques ;
b. placer le composant magnétique externe médian dans la partie médiane dudit corps de coque ;
c. placer et fixer une plaque médiane ;
d. insérer deux plaques de blindage magnétique de part et d'autre du composant magnétique externe médian pour blinder le champ magnétique ;
e. installer le composant magnétique externe gauche et le composant magnétique externe droit ;
f. placer et fixer une plaque droite et une plaque gauche, lesdites plaque gauche et plaque droite étant réalisées en matériaux non ferromagnétiques ; et
g. retirer les deux plaques de blindage magnétique.

21. Procédé selon la revendication 20, dans lequel lesdites plaques de blindage magnétique sont réalisées en permalloy.
